# EUROPEAN PATENT APPLICATION

(11) **EP 2 907 502 A1**
(43) Date of publication of application: **19.08.2015**
(21) Application number: 15154624.9
(22) Date of filing: 11.02.2015
(51) Int. Cl.: A61K 9/00, A61K 47/34, A61L 15/22, A61L 15/26, A61L 15/42, A61L 15/44, A61L 15/46, C08L 75/04

(54) **Interface membrane for the contact with the skin**

(30) Priority: 17.02.2014 IT MI20140230
(71) Applicant: Ferrari S.r.l., 25020 Bassano Bresciano (Brescia) (IT)
(72) Inventor: Caba, Valentina, I-25018 MONTICHIARI (Brescia) (IT)
(74) Representative: Lunati & Mazzoni S.r.L.

(57) **Abstract**

A production method (10) is provided for of an interface membrane (1) for contact with the skin, suitable to be connected to a support layer (4) and to be placed in contact with the human body, the interface membrane (1) having a structure with a main two-dimensional extension and defining a first surface (1a) suitable to come into contact with the skin, the membrane (1) comprising at least one layer (3) defining the first surface (1a) and made in crosslinking polyurethane foam, the method comprising at least one coating step (20) of the polyurethane foam on a transfer medium (20a), to make said at least one layer (3), and a detachment step (45) of said interface membrane (1) from said transfer medium (20a), said transfer medium (20a) having a roughness equivalent to a transfer paper with less than 4% Gloss, measured using the method indicated, 75° TAPPI T480".

## Description

The present invention relates to an interface membrane for the contact with the skin of the type described in the preamble of claim 1.

In particular, the invention relates to an interface membrane for contact with the skin suitable to be placed in contact with the skin and preferably a further support such as a brace, bandage or the like and, in addition, preferably, to release one or more active substances which are thus absorbed through the skin barrier. Currently similar medical devices, such as transdermal patches, normally consist of two layers: one active layer containing the active substance; and a protection composed of a sheet impermeable to the active substance and to water, with a support and protection function for the preparation.

The active layer is composed of a single layer or multi-layer solid or semi-solid matrix identifying a reservoir of active substance and suitable to permit the diffusion of the active substance to the skin. In addition, the active layer in order to optimise the release, may comprise a membrane which controls the release and diffusion of the active substance from the matrix.

Lastly, the active layer has a contact surface adhesive on the skin to allow the membrane to stick firmly to the skin and, at the same time, to remove the membrane without causing damage to the skin or detachment of the preparation from the outer protection.

The prior art mentioned above has several significant drawbacks.

A first drawback is the fact that interface membranes for contact with the skin have reduced breathability and may therefore lead to irritation of the skin or to other similar problems in the area of application of the patch.

Consequently, an important drawback is identifiable in the fact that the known membranes can only be applied for a short time and, therefore, do not permit optimal use of the support or of the active substance.

Another drawback is represented by the fact that the known membranes are particularly inflexible and rigid so that they are uncomfortable to wear.

In particular, the membrane being inflexible, is unable to adapt to the movements of skin and, on account of the adhesive layer, pulls the skin causing a feeling of discomfort to the user.

A further drawback, caused by said reduced flexibility lies in the fact that the known devices do not adhere well to the curves of the body.

As a result, the interface membranes for contact with the skin used up until now do not ensure contact between the active layer and the skin and, thus, the proper absorption of the active substance by the skin.

This drawback is particularly evident if the membrane is applied at the joints or other similar parts of the body, where, due to continuous movements the membrane incurs in detachments which prevent the support of the brace or release of the active substance.

To resolve this drawback, some interface membranes for contact with the skin have an outer layer in contact with the skin provided with glue which, on the one hand, ensures a high adhesion but which, on the other, makes the detachment of the membrane particularly painful and, moreover, interposes itself between the membrane and the skin, limiting the release of active substances.

In this situation the technical purpose of the present invention is to devise a production method of an interface membrane for contact with the skin able to substantially overcome the drawbacks mentioned.

Within the sphere of said technical purpose one important purpose of the invention is to have an interface membrane for contact with the skin characterised by elevated breathability and, thus, suitable to be applied to a given point for long periods.

Another important aim of the invention is to obtain an interface membrane for contact with the skin which is comfortable to wear.

A further aim of the invention is to devise an interface membrane for contact with the skin, which is suitable to remain virtually perfectly adherent to the skin independently of the movements of the user. A further aim of no less importance of the present invention is to identify a production method which permits a great versatility in the production of the membrane making it possible, for example, to change the active substance, the elasticity of the membrane or its use.

In particular, an important aim is therefore to have a method that makes it possible to produce a customizable membrane in virtually all its components.

The technical task and specified aims are achieved by a production method of an interface membrane for contact with the skin according to annexed claim 1. Preferred embodiments are evident from the dependent claims.

The characteristics and advantages of the invention are clearly evident from the following detailed description of a preferred embodiment thereof, with reference to the accompanying drawings, in which:
**Fig. 1** shows a portion of cross-section of the membrane according to the invention;
**Fig. 2** shows a diagram of the operation of a production plant of the membrane according to the invention; and
**Fig. 3** shows a production method of the membrane according to the invention.

With reference to said Drawings, the interface membrane for contact with the skin according to the invention is globally denoted by reference numeral **1.**

It is suitable to be combined with a support layer **5** described below in detail, to make a brace **6,** in particular a bandage, a rigid support or otherwise.

The membrane 1 preferably has a tape structure with a main two-dimensional extension and defines a first surface **1a** suitable to come into contact with the skin and an outer surface **1b,** opposite the first surface and suitable to be connected to the support layer 5.

The membrane 1 preferably comprises at least one layer 3 advantageously made of a crosslinking polyurethane foam i.e. in a foam of polyurethane resins with crosslinking agents added, said layer 3 defining at least said first surface 1 a. Appropriately, said foam of polyurethane resins comprises a mixture of polyurethane and air suitable to create an open cell foam.

The crosslinking agent is preferably an isocyanate.

More preferably, the layer 3 is in a crosslinking foam preferably with one or more active substances **2** added, described below and, additionally, pigments.

The membrane 1 preferably has a thickness comprised between 1 mm and 1 cm.

In particular, the active substances 2 may comprise active ingredients or other elements useful for medical-therapeutic purposes or simply rehabilitation. These comprise, for example, sanitising agents suitable to ensure the anti-bacterial properties of said membrane 1. The antibacterial agents are also suitable to act on possible bacterial strains and are suitable to deposit themselves on the membrane 1, thereby eliminating the risk of infections, mildew and foul odours. They can be analysed in advance to ensure the total absence of cytotoxicity.

Lastly, the antibacterial agents, being included in the formula together with the polyurethane resin, bind to it by means of chemical bonds and thereby become one with the resin.

Moreover, the active substances 2 may comprise PCM (phase change particles, or phase-change material) appropriately embedded in the polyurethane foam and suitable to absorb or release heat so as to maintain the surface body temperature which they come in contact with substantially constant.

In detail, the PCM is characterised by a solidification temperature substantially equal to the body temperature, preferably of about 35°C. It is of the organic type and, more specifically, is paraffin.

The membrane 1 is preferably composed of a plurality of layers 3, all of the same material, preferably there are three 3 layers: a first layer **3a,** defining the first surface 1a suitable to come into contact with the skin, a third layer **3c,** defining the outer surface 1 b, and a second layer **3b,** interposed between the first layer 3a and the third layer 3c.

Said layers 3 have a thickness substantially identical to each other, are preferably made of the same material and, once made do not exhibit substantial discontinuities between them.

The support layer 5 is preferably a fabric. In particular, the covering 5 is made of rigid fabric, such as, for example Velcro fabric or, alternatively, elastic fabric. Alternatively, the support 5 may be a rigid or flexible membrane.

In order to connect the support layer 3c, in correspondence with the outer surface 1 b to one of the layers 3 and, in particular, to the third layer 3c, the membrane 1 has an adhesive layer **4** suitable to connect the support layer 5 to one of the layers and, in particular, to the third.

The adhesive layer 4 comprises a crosslinking polyurethane resin appropriately suitable for adding smoothing agents to.

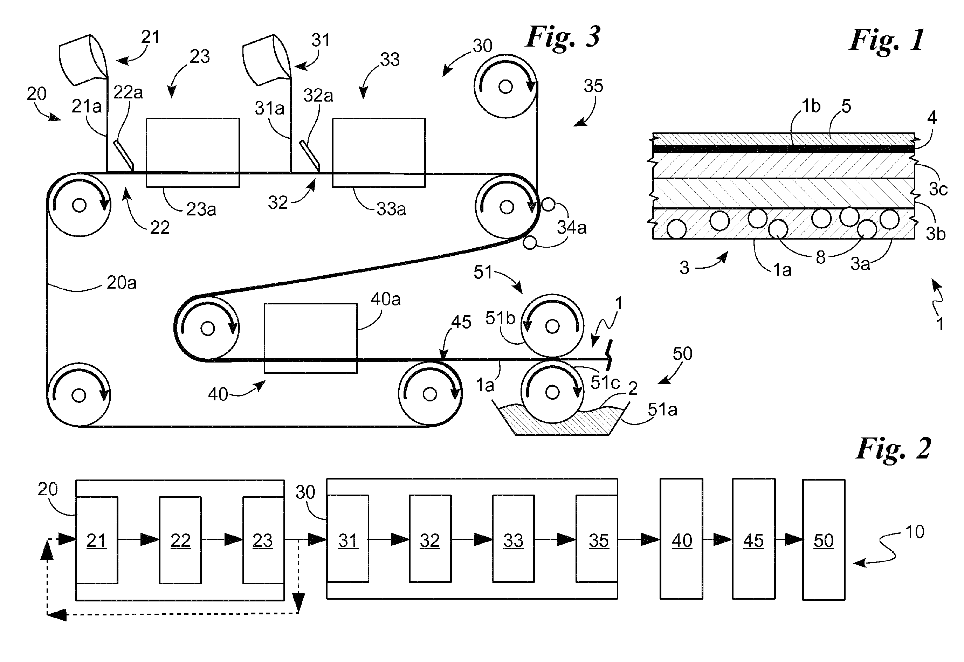

The membrane 1 is obtained by means of a production method shown schematically in Fig. 1 and indicated by reference numeral **10.**

The method 10 preferably comprises: a coating step **20** in which at least one layer 3 is coated; a second step of coating **30** in which the adhesive layer 4 is spread; a crosslinking step **40** of at least the first layer 3; and a step of depositing **50** in which one or more active substances 2 are deposited on the first surface 1a and absorbed by at least the first layer 3a and, in detail by all the layers 3a, 3b and 3c. Appropriately, the production method 10 preferably comprises at least two coating steps 20 suitable to be carried out sequentially so as to deposit the outer layer or third layer 3c over the first layer 3a. More appropriately, the method 10 provides for three coating steps 20 so as to deposit the first layer 3a, and then, the second layer 3b on the first layer 3a and, lastly, the third layer 3c on the second layer 3b. In the coating step 30, the polymer, in particular the polyurethane, in the liquid state and/or still in formation, is spread on a transfer medium **20a** as described below.

Each coating step 20 comprises a sub-step of preparation of the polymeric material, a deposit sub-step **21** in which a polyurethane foam **21a** is deposited, preferably, by gravity, on a transfer medium **20a,** a spreading sub-step **22** in which said polyurethane foam 21 a is spread on the transfer medium 20a; and a drying sub-step **23** of the polyurethane solution 21 a.

The transfer medium 20a is preferably a flexible membrane, preferably made of transfer paper, known per se, or in other materials, for example polymer. Appropriately it has a matt and very smooth surface, and preferably a weight of 100 g/m² and 300 g/m².

The transfer medium 20a gives the polymer the proper surface roughness, which achieves the correct contact between the first layer 3a and the skin of the user, as described in detail below.

In particular the transfer medium 20a has a roughness equivalent to a transfer paper with less than 4% and preferably more than 1% Gloss, measured using the 75° TAPPI T480" method. This method is known per se to experts in the sector of the transfer paper, and thus of the coatings on transfer paper. It can be found for example on the website www.tappi.org. TAPPI in fact stands for "Technological Association of the Pulp and Paper" present since 1915. The transfer medium 20a may obviously be said transfer paper with the roughness described.

The polyurethane foam 21 a comprises the polyurethane resins forming said polyurethane foam and a solvent, appropriately water, suitable to be eliminated by evaporation in the drying sub-step 23 and, if provided for, PCM 7 and the crosslinking agents, sanitising agents and pigments.

In particular, the polyurethane foam 21 a is made, in the preparation sub-step starting from a polyurethane, preferably of the closed type, which has therefore already undergone the reaction of polyols - isocyanates, which is also preferably soft, i.e. in particular with a modulus between 10 and 150 kg/cm² according to the standard DIN53504. Subsequently a number of additives may be added to said polyurethane such as: a foaming agent, to create foam, a crosslinking agent to improve the resistance to solvents and hydrolysis, as described below, a possible pigment, only for possible colouring and thickeners. Said elements are known per se. Subsequently, the polyurethane preferably with additives is mixed with water. The water mass is preferably between 40% and 80% of the weight of the polyurethane, and more preferably between 60% and 70%.

The polyurethane preferably with additives and mixed with water is mechanically foamed, i.e. agitated with rotating beaters and the like, to obtain a density between 200 and 700 grams/litre.

In the spreading sub-step 22, the polyurethane foam, moved by the transfer medium 20a, encounters a regulation blade **22a,** placed next to the transfer medium 20a and substantially parallel to the transfer medium 20a, suitable to evenly distribute the foam along the membrane 20a making a layer 3 of a uniform thickness and substantially equal to the distance between the blade 22a and transfer medium 20a; and a regulator suitable to vary the distance of the regulation blade 22a from the transfer medium 20a and thus, the thickness of the layers 3.

Once past the blade 22a, the transfer medium 20a introduces the polyurethane foam inside a drying oven **23a** in which the drying sub-step 23 takes place.

In detail, the drying oven 23a is suitable to remove the solvent from the foam by evaporation, obtaining the polyurethane foam by bringing the foam to a temperature between 100°C and 180°C and for a time ranging from 60 seconds to 300 seconds.

After completing the production of the layers 3a, 3b and 3c, the membrane 1, still moved by the transfer medium 20a, is subjected to the gluing step 30.

This second coating step 30 comprises an additional depositing sub-step **31** in which an additional polyurethane mixture **31 a** is deposited, on the outer surface 1b preferably by gravity, on one of the layers 3 and, in particular, on the third layer 3c; an additional spreading sub-step **32** in which an additional blade **32a** spreads the additional mixture ensuring its even distribution all over the surface of the third layer 3c; an additional drying sub-step **33** in which the additional polyurethane mixture is dried 31 a.

The second coating step 30 may be followed by an attachment step **35** wherein the coating 5 is pressed against the adhesive layer 4, for example by means of heated pressure rollers **35a,** thus connecting itself to the rest of the membrane 1.

The additional polyurethane mixture 31 a comprises the polyurethane resins forming the adhesive layer 4, if provided for, the smoothing agents and a solvent, appropriately water, suitable to be removed by evaporation in the additional drying sub-step 33.

Once the gluing step 30 is concluded, the production method preferably comprises the crosslinking step 40 in which the transfer medium 20a brings the membrane 1 inside a crosslinking oven **40a.**

The drying oven 40a is suitable to heat the layers 3 and 4 so as to cause their crosslinking. It is therefore suitable to heat the membrane 1 to a temperature substantially between 100°C and 180°C for a period of time between about 60 seconds and 300 seconds.

Following said steps a detachment step **45** of the membrane 1 from the transfer medium 20a is carried out in which the transfer medium 20a is moved by a movement apparatus suitable to place the first surface 1a in view, permitting the performance of the depositing step 50 in which one or more roller deposit devices **51,** preferably three, deposit one or more active substances 2 on the first surface 1 a. Each roller deposit device 51 comprises a draught tank **51a** designed to contain one or more active substances 2; a movement roller **51 b** suitable to move the membrane 1 placing the first surface 3a in view; a deposit roller **51c** suitable to work and rotate in opposition to the movement roller 51 b so as to press the membrane 1 against the movement roller 51 b and at least partially immersed in the tank 51 a to withdraw the active substances 2 from the tank 51 a and deposit them on the surface 3a.

The invention achieves some important advantages.

A first important advantage is that the membrane 1 is characterised by a high degree of breathability and can therefore be used for an extended period of time without the risk of irritation or other similar problems in the application area of the membrane 1.

Said aspect has been innovatively achieved thanks to the adoption of layers 3a, 3b and 3c made of polyurethane foams and, in particular, crosslinking polyurethane foams.

In addition, the adoption of the crosslinking agent and thus of crosslinking polyurethane foams makes it possible to have a membrane with high resistance to alcohol or other substances usually applied to the area where the membrane is attached and/or used in combination with a membrane.

In fact, the crosslinking agent, intervenes to strengthen the bonds of the polyurethane, such crosslinking agent may be an isocyanate.

Another advantage is that the adoption of crosslinking polyurethane foam layers 3 allows the membrane 1 to be very flexible and, therefore, suitable to ensure a perfect contact between the first surface 1a and the skin regardless of the area of application and of movements of the skin, without being at the same time overly adherent or spread on the skin.

Such aspect is further incremented by the proper roughness innovatively selected of the transfer medium 20a.

In addition, optimal contact with the skin is ensured thanks to the fact that the polyurethane foams and, in particular, the crosslinking polyurethane foams are characterised by a high grip capacity and thus bind tightly to the skin without the need for glues on the first surface 1 a.

Such aspect makes it possible to have, in every point, the first surface 3a in direct contact with the skin and, thus, to increase the release capacity of the active substances 2 compared to the known devices of the same size.

One important advantage is that the membrane 1 is much more effective than the known devices.

An additional advantage is the presence of PCM which, being characterised by a solidification temperature almost equal to that of the body, maintains the surface body temperature practically constant and thus protects it from traumas caused by thermal shocks.

Another advantage is provided by the adoption of an open-cell foam which ensures high breathability and softness of the membrane 1.

The invention is susceptible to variations within the inventive concept. All the elements as described and claimed may be replaced with equivalent elements and the details, materials, shapes and dimensions may be as desired.

## Claims

1. Production method (10) of an interface membrane (1) for contact with the skin, suitable to be connected to a support layer (4) and to be placed in contact with the human body,
- said interface membrane (1) having a structure with a main two-dimensional extension and defining a first surface (1a) suitable to come into contact with the skin,
- said membrane (1) comprising at least one layer (3) defining said first surface (1 a) and made in crosslinking polyurethane foam,
- said method being **characterised in that** it comprises:
- at least one coating step (20) of said polyurethane foam on a transfer medium (20a), to make said at least one layer (3), and
- a detachment step (45) of said interface membrane (1) from said transfer medium (20a)
and **in that** said transfer medium (20a) has a roughness equivalent to a transfer paper with less than 4% Gloss measured using the method: "75° TAPPI T480".

2. Production method (10) according to previous claim, wherein said transfer medium (20a) has a roughness equivalent to a transfer paper with more than 1% Gloss, measured using the method indicated, 75° TAPPI T480".

3. Production method (10) according to one or more of the previous claims, wherein said transfer medium (20a) is transfer paper.

4. Production method (10) according to one or more of the previous claims, comprising a preparation sub-step, in which said polyurethane foam (21a) is prepared starting from a polyurethane and additives such as: a foaming agent, a crosslinking agent, and wherein said polyurethane is subsequently mixed with water and mechanically foamed.

5. Production method (10) according to the previous claim, wherein said polyurethane has a modulus between 10 and 150 kg/cm² according to the standard DIN53504.

6. Production method (10) according to claims 3 or 4, wherein said polyurethane is mechanically foamed until a density between 200 and 700 grams/litre is achieved.

7. Production method (10) according to one or more of the preceding claims, wherein said interface membrane (1) comprises a plurality of said layers (3), each made with a single said coating step (20).

8. Production method (10) according to the preceding claim, wherein said interface membrane (1) comprises three of said layers (3).

9. Production method (10) according to one or more of the preceding claims, wherein each said coating step (20) comprises a deposit sub-step (21) in which said polyurethane foam (21 a) is deposited on said transfer medium (20a) and a spreading sub-step (22) in which said polyurethane foam (21 a) is spread on said transfer medium (20a).

10. Production method (10) according to the preceding claim, wherein said coating step (20) comprises a sub-step of drying (23) said polyurethane foam (21 a).

11. Production method (10) according to one or more of the preceding claims, comprising a second coating step (30) of an adhesive layer (4) on said surface (3) on an outer surface (1 b) opposite said first surface (1 a).

12. Production method (10) according to the preceding claim, wherein said second coating step (30) comprises an additional deposit sub-step (31) in which an additional polyurethane mixture (31 a) is deposited, on the outer surface (1b); an additional spreading sub-step (32) in which an additional blade (32a) spreads the additional mixture and an additional drying sub-step (33) in which said additional polyurethane mixture (31 a) is dried.

13. Production method (10) according to one or more of the preceding claims, comprising a deposit step (50) in which at least one active substance (2) is deposited on the first surface(1 a) and absorbed by at least one of said layers (3).

14. Production method (10) according to the preceding claim, wherein one of said active substances (2) comprises PCM.

15. An interface membrane (1) for the contact with the skin, suitable to be connected with a support layer (4) and to be placed in contact with the human body, realized by the production method (10) according to one or more of the preceding claims.
